# EUROPÄISCHE PATENTSCHRIFT

(11) **EP 3 334 974 B1**
(45) Veröffentlichungstag und Bekanntmachung des Hinweises auf die Patenterteilung: **09.10.2019**
(21) Anmeldenummer: 16751515.4
(22) Anmeldetag: 25.07.2016
(51) Int. Cl.: F21V 21/40, F21V 23/04

(54) **HANDGRIFFVORRICHTUNG FÜR EINE OPERATIONSLEUCHTE MIT SENSOREN SOWIE OPERATIONSLEUCHTE**
HANDLE DEVICE FOR A SURGICAL LIGHT WITH SENSORS, AND SURGICAL LIGHT
DISPOSITIF DE POIGNÉE POUR UNE LAMPE DE SALLE D'OPÉRATION AVEC DES CAPTEURS AINSI QUE LAMPE DE SALLE D'OPÉRATION

(30) Priorität: 13.08.2015 DE 102015113336
(43) Veröffentlichungstag der Anmeldung: 20.06.2018
(73) Patentinhaber: Karl Leibinger Medizintechnik GmbH & Co. KG, 78570 Mühlheim/Donau (DE)
(72) Erfinder: STRÖLIN, Joachim, 78604 Rietheim (DE)
(74) Vertreter: Winter, Brandl, Fürniss, Hübner, Röss, Kaiser, Polte - Partnerschaft mbB
(86) Internationale Anmeldenummer: PCT/EP2016/067689
(87) Internationale Veröffentlichungsnummer: WO 2017/025308

(56) Entgegenhaltungen:
- EP-A1- 2 434 202
- CN-A- 1 363 796
- US-A1- 2014 268 751

## Beschreibung

Die Erfindung betrifft eine Handgriffvorrichtung für eine Operationsleuchte, mit einem, zum Anbringen an einem Leuchtenaufnahmekörper der Operationsleuchte vorbereiteten Griffelement (auch als Handgriff oder schlicht als Griff bezeichnet), welches Griffelement an einem Außenbereich eine Grifffläche ausbildet. Die Erfindung betrifft auch eine Operationsleuchte mit einer solchen Handgriffvorrichtung.

Aus dem Stand der Technik ist es bisher allgemein bekannt, bei Operationsleuchten eine gewisse Sensorik vorzusehen, die ein Objekt, das sich zwischen dem zu operierenden Bereich / dem Wundfeld und dem Leuchtenaufnahmekörper der Operationsleuchte befindet, detektiert. Auch ist es in diesem Zusammenhang bekannt, dass, wenn das Objekt, etwa ein Körperteil (z.B. Kopf) einer Person die von einzelnen Leuchten / Einzelleuchten der Operationsleuchte erzeugten Lichtstrahlbündel teilweise verdeckt, die verdeckten Einzelleuchten in ihrer Helligkeit / Beleuchtungsstärke abzuschwächen oder gar abzuschalten. Die übrigen, nicht durch das Objekt verdeckten Einzelleuchten der Operationsleuchte können dabei gleichzeitig erhellt werden, um dennoch eine gleichmäßig starke Ausleuchtung des Wundfeldes zu ermöglichen. Bei diesen Operationsleuchten sind die Sensoren zumeist direkt innerhalb des Leuchtenaufnahmekörpers der Operationsleuchte eingebaut.

Somit ist es bereits möglich, die Intensität der Lichtstrahlung der Operationsleuchte und die dadurch verbundene Wärmeentwicklung auf dem Objekt, etwa dem Kopf eines Operateurs zu reduzieren oder gar zu vermeiden. Von Nachteil hat es sich jedoch bei diesen bekannten Systemen herausgestellt, dass die mit der Sensorik bestückten Operationsleuchten zumeist aufgrund des erhöhten Herstellungsaufwandes, relativ aufwändig und somit auch kostenintensiv herzustellen sind. Auch wegen der noch relativ geringen Anzahl an Kunden, die eine solche Sensorik überhaupt wünschen, war es bisher relativ unwirtschaftlich diese mit der Sensorik fest verbundenen Operationsleuchten in hoher Stückzahl zu produzieren. Insbesondere für jene Endverbraucher, die an dieser Sensorik primär nicht interessiert sind, ist eine solche Operationsleuchte häufig zu teuer.

Weiterer Stand der Technik ist mit der EP 2 434 202 A1, der CN 1 363 796 A sowie der US 2014/268751 A1 bekannt.

Es ist somit die Aufgabe der vorliegenden Erfindung, diese aus dem Stand der Technik bekannten Nachteile zu beheben und eine Operationsleuchte zu ermöglichen, die in ihrer Herstellung in hohen Stückzahlen besonders profitabel ist, jedoch individuell mit einer Sensorik nachrüstbar sein soll.

Dies wird erfindungsgemäß durch den Gegenstand des Anspruchs 1 gelöst. Dabei ist eine Handgriffvorrichtung vorgesehen, wobei mit deren Griffelement ein Sensormodul wieder abnehmbar verbunden ist und das Sensormodul zumindest einen Abstandssensor aufweist, der zum Detektieren einer Position eines Objektes (d.h. zum Messen eines Abstandes zwischen dem Objekt und der Handgriffvorrichtung / dem Sensormodul) ausgelegt ist. Mehrere Abstandssensoren sind in dem Sensormodul vorhanden, deren Messfelder / Wirkbereiche jeweils mit ihrer Mittelachse / Richtachse (von dem Gehäuse aus) voneinander weg gerichtet sind.

Durch diesen modularen Aufbau ist es möglich, wenn der Endverbraucher einer Operationsleuchte zusätzlich eine Sensorik wünscht, die bestehende Handgriffvorrichtung einfach durch eine Handgriffvorrichtung der erfindungsgemäßen Art auszutauschen. Denn die bekannten Handgriffvorrichtungen sind ohnehin zumeist austauschbar mit dem Leuchtenaufnahmekörper verbunden, um sie zur Sterilisation abzunehmen.

Dadurch kann die Operationsleuchte individuell mit dem, Abstandssensoren aufweisenden Sensormodul besonders einfach nachgerüstet werden. Auch ist eine möglichst umfangreiche Erfassung des Bereiches unterhalb des Leuchtenaufnahmekörpers im Betriebszustand umgesetzt.

Weitere vorteilhafte Ausführungsformen sind in den Unteransprüchen beansprucht und nachfolgend näher erläutert.

Weiterhin vorteilhaft ist es, wenn das Sensormodul ein Gehäuse aufweist, in dem die Abstandssensoren aufgenommen / angebracht / befestigt sind. Dadurch ist eine möglichst robuste Ausführung des Sensormoduls ausgebildet und der Abstandssensor vor der Umgebung geschützt.

In diesem Zusammenhang ist es auch weiterhin von Vorteil, wenn das Gehäuse einen scheibenförmigen Hohlkörperabschnitt aufweist / ausbildet, innerhalb dessen die Abstandssensoren angeordnet / befestigt sind. Dadurch ist das Sensormodul besonders kompakt ausgestaltet und nimmt möglichst wenig Bauraum ein.

Ist das Gehäuse im Bereich der Abstandssensoren durchlässig für ein durch die Abstandssensoren zu detektierendes Messsignal (vorzugsweise Infrarotlicht-durchlässig) ausgebildet, sind die Abstandssensoren besonders vorteilhaft, geschützt von der Umgebung, in dem Gehäuse aufgenommen.

Ist zudem der zumindest eine Abstandssensor als ein Infrarotsensor ausgebildet, ist das Sensormodul mit bewährten Messsensoren ausgestattet, die eine noch kostengünstigere Herstellung des Sensormoduls / der Handgriffvorrichtung ermöglichen.

Zweckmäßig ist es auch, wenn das Sensormodul einen Handgriffaufnahmeabschnitt aufweist, der wiederabnehmbar mit dem Griffelement verbunden ist. Dadurch ist ein unsteril gemachtes Griffelement durch ein neues / steriles Griffelement besonders einfach auszutauschen, ohne dabei das Sensormodul mit abnehmen zu müssen. Zudem ist es dadurch ermöglicht, verschiedene Griffelemente mit dem Sensormodul zu verbinden.

In diesem Zusammenhang ist es auch von Vorteil, wenn der Handgriffaufnahmeabschnitt einen Aufnahmezapfen ausbildet, auf dem in zumindest einem Betriebszustand der Handgriffvorrichtung ein, die Grifffläche aufweisender Hülsenabschnitt des Griffelementes aufgesteckt / aufgeschoben, d.h. formschlüssig aufgebracht ist. Dadurch ist eine besonders stabile Halterung des Griffelementes relativ zu dem Sensormodul umgesetzt.

In diesem Zusammenhang ist es auch vorteilhaft, wenn das Griffelement form- und/oder kraftschlüssig mit dem Sensormodul in zumindest einem Betriebszustand verbunden ist, da dadurch der Austausch des Griffelementes besonders rasch erfolgen kann.

Vorteilhaft ist es im Weiteren, wenn das Sensormodul eine Rechnereinheit (auch als Recheneinheit bezeichnet) aufweist, die elektrisch mit den Abstandssensoren verbunden sind und in Abhängigkeit von den mittels der Abstandssensoren ermittelten Messdaten Steuersignale zur Steuerung der Operationsleuchte erzeugt. Dadurch ist eine besonders direkte elektrische Anbindung / Versorgung der Abstandssensoren umgesetzt. Im Betrieb wird die Operationsleuchte in Abhängigkeit dieser Steuersignale dann derart unmittelbar angesteuert, dass die entsprechenden, das Objekt / den Operateur bestrahlenden Leuchtelemente / Einzelleuchten herunter gedimmt oder gar ausgeschaltet werden können.

Vorteilhaft ist es im Weiteren, wenn in dem Sensormodul eine Sprachsteuerungseinheit zum Betätigen der Abstandssensoren und/oder zum Steuern der Operationsleuchte in zumindest einem Betriebszustand, integriert ist. Die Sprachsteuerungseinheit, die weiter bevorzugt zumindest ein Mikrofon, d.h. einen Schallsensor aufweist, ermöglicht es, die einzelnen Leuchten der Operationsleuchte individuell zu steuern. Auch können mittels dieser Sprachsteuerungseinheit noch zahlreiche weitere Funktionen umgesetzt sein und etwa Steuersignale erzeugt werden, die die Höheneinstellung oder die Neigung der Operationsleuchte verändern. Dadurch ist es möglich, im Betrieb einen direkten Kontakt des Operateurs mit der Operationsleuchte zu vermeiden.

In diesem Zusammenhang ist es weiterhin auch zweckmäßig, wenn die Sprachsteuerungseinheit elektronisch mit der Recheneinheit / Rechnereinheit verbunden ist. Dadurch wird die Sprachsteuerungseinheit unmittelbar mit der ohnehin vorhandenen Rechnereinheit zur Auswertung der Messdaten der Abstandssensoren eingesetzt. Der Aufbau des Sensormoduls wird relativ einfach gehalten, womit eine noch kostengünstigere Herstellung umgesetzt ist.

Im Weiteren umfasst die Erfindung auch eine Operationsleuchte mit einer Handgriffvorrichtung nach zumindest einer der zuvor genannten Ausführungsformen, wobei die Handgriffvorrichtung zumindest teilweise wieder abnehmbar mit einem Leuchtenaufnahmekörper der Operationsleuchte verbunden ist, wodurch die Sterilisierbarkeit der Handgriffvorrichtung besonders einfach umgesetzt ist.

In diesem Zusammenhang ist es auch von Vorteil, wenn das Sensormodul wieder abnehmbar an dem Leuchtenaufnahmekörper angebracht ist, da dann das Sensormodul bei Bedarf rasch von der Operationsleuchte nach einem vorhergehenden Befestigen, entfernt werden kann. Dadurch ist die Operationsleuchte besonders individuell ausrüstbar.

Ist eine Recheneinheit des Sensormoduls elektrisch mit einer zentralen Steuereinheit der Operationsleuchte verbunden, können Steuersignale von dem Sensormodul direkt auf die Operationsleuchte übertragen werden, wodurch die Operationsleuchte ihre einzelnen Leuchten (insbesondere hinsichtlich ihrer Beleuchtungsstärke / Helligkeit) individuell und direkt einstellen kann. Die Ansteuerung der Operationsleuchte ist somit möglichst direkt umgesetzt.

Erfindungsgemäß ist somit eine Sensorik mit Abstandssensoren ausgestattet, die in der zentralen Handgriffeinheit / Handriffvorrichtung der Operationsleuchte untergebracht sind. Diese zentrale Handgriffeinheit ist an sich leicht austauschbar, wodurch eine Operationsleuchte sehr einfach und schnell mit den Sensoren, auch bei Nachkauf nachgerüstet werden kann. Es gibt dabei unterschiedliche Varianten an Handgriffsgruppen / Handriffvorrichtungen, wobei bspw. eine ohne Sensoren und eine mit Sensoren (gemäß der Erfindung) vorhanden ist, die dann einfach ausgetauscht werden können. In einer weiteren Variante ist das Sensormodul als ein Zwischenring zwischen der Handgriffgruppe und dem Leuchtenaufnahmekörper eingebaut. Durch die zentrale Anordnung der Sensoren und der zentralen Handgriffeinheit in der Operationsleuchte ist somit eine optimale "Sicht", der Sensoren auf die Köpfe der Operateure / Anwender ermöglicht. Die Sensoren erfassen den Kopf als Hindernis und blenden daraufhin die dahinterliegenden Teilbereiche aus. Die Sensoren sind auf einer ringförmigen Platine bestückt. Das runde / konische Bauteil (Seitenwand des Gehäuses / des Hohlkörperabschnittes) ist transparent, nämlich IR-durchlässig, ausgeführt / ausgebildet.

Die Erfindung wird nun nachfolgend anhand von Figuren näher erläutert, in welchem Zusammenhang auch verschiedene Ausführungsbeispiele beschrieben sind.

Es zeigen:
- Fig. 1: eine isometrische Darstellung einer erfindungsgemäßen Handgriffvorrichtung nach einem ersten Ausführungsbeispiel, in der ein Griffelement fest mit einem Sensormodul verbunden ist,
- Fig. 2: eine isometrische Darstellung der Handgriffvorrichtung aus Fig. 1, wobei nun lediglich das Sensormodul dargestellt ist und das Griffelement von einem Aufnahmezapfen des Sensormoduls abgenommen ist, und wobei die Sprachsteuerungseinheit hinter der leicht transparent dargestellten Außenwandung des Gehäuses zu erkennen ist,
- Fig. 3: eine isometrische Darstellung eines Teils des Sensormoduls nach Fig. 2, wobei das Innere des Gehäuses, in dem die die Aufnahmesensoren aufnehmende Platine angebracht ist, ersichtlich ist,
- Fig. 4: eine schematische Seitenansicht der Handgriffvorrichtung nach Fig. 1, wobei die Anordnung zwischen dem Gehäuse / Sensormodul und dem Griffelement zu erkennen ist,
- Fig. 5: eine Seitenansicht einer erfindungsgemäßen Operationsleuchte nach einem ersten vorteilhaften Ausführungsbeispiel, wobei die Handgriffvorrichtung wie schon in Fig. 4 schematisch dargestellt ist und an einem Leuchtenaufnahmekörper der Operationsleuchte befestigt ist,
- Fig. 6: eine Unteransicht der in Fig. 5 dargestellten Operationsleuchte, wobei besonders gut die verschiedenen, jeweils eine Mehrzahl an Einzelleuchten aufweisenden Leuchtenfelder der Operationsleuchte, die im Betrieb das Wundfeld beleuchten, zu erkennen sind,
- Fig. 7: eine schematische Seitendarstellung einer Operationsleuchte mit zwei unterschiedlichen, in einem demontierten Zustand befindlichen Handgriffvorrichtungen, wobei die rechte der beiden dargestellten Handgriffvorrichtungen jene Handgriffvorrichtung der erfindungsgemäßen Art nach Fig. 1 bzw. 4 ist, und die linke der beiden Handgriffvorrichtungen eine aus dem Stand der Technik bekannte Handgriffvorrichtung ist, welche Handgriffvorrichtungen beide an den dargestellten Leuchtenaufnahmekörpern der Operationsleuchte befestigbar sind,
- Fig. 8: eine Operationsleuchte gemäß des Standes der Technik, d.h. mit einer Handgriffvorrichtung ohne dem erfindungsgemäßen Sensormodul,
- Fig. 9: eine schematische Seitenansicht einer erfindungsgemäßen Operationsleuchte nach den Fign. 5 und 6, wobei besonders gut ein im Betrieb erzeugter Wirkbereich eines Abstandssensors des Sensormoduls zu erkennen ist, und wobei sich im Wirkbereich des Abstandssensors ein Objekt, nämlich ein Kopf einer Person befindet,
- Fig. 10: eine schematische Seitenansicht eines Sensormoduls für eine Handgriffvorrichtung nach einem vorteilhaften weiteren Ausführungsbeispiel, wobei das Sensormodul nun ringförmig ausgebildet ist und keinen Aufnahmezapfen aufweist,
- Fig. 11: eine schematische Unteransicht der erfindungsgemäßen Operationsleuchte, wobei wie in Fig. 9 innerhalb der Reichweite des Sensormoduls bzw. deren Abstandssensoren ein rundes Objekt, wie mit dem Pfeil gekennzeichnet, in die Lichtstrahlbündel einzelner Leuchtenfelder hinein bewegt wird, woraufhin die dunkel dargestellten Einzelleuchten der Leuchtenfelder aufgrund der detektierten Position mittels zumindest eines Abstandssensors selbsttätig mithilfe einer Rechnereinheit abgedimmt werden,
- Fig. 12: eine Unteransicht der Operationsleuchte gemäß Fig. 11, wobei das Objekt nun weiter in Richtung des zentralen Handgriffs / der zentralen Handgriffvorrichtung bewegt ist und gegenüber Fig. 11 weitere Einzelleuchten der Leuchtenfelder abgeschaltet sind,
- Fig. 13: wiederum eine Unteransicht der Operationsleuchte, gemäß Fign. 11 u. 12, wobei das Objekt gegenüber Fig. 12 noch weiter in Richtung des Zentrums, d.h. in Richtung der Handgriffvorrichtung bewegt ist und daraufhin noch weitere Einzelleuchten der Operationsleuchte abgedimmt / ausgeschaltet sind,
- Fig. 14: eine Innenseite, d.h. eine dem Innenraum des Hohlkörperabschnittes zugewandte Seite, der in Fig. 3 bereits dargestellten Platine des Sensormoduls,
- Fig. 15: eine isometrische Detailansicht der Platine aus Fig. 14 im Bereich zweier benachbarter Abstandssensoren, wobei deren Anzeigeleuchten gut zu erkennen sind,
- Fig. 16: eine isometrische Detailansicht einer Platine einer Handgriffvorrichtung gemäß einem weiteren vorteilhaften Ausführungsbeispiel, wobei deren Rechnereinheit gut zu erkennen ist,
- Fig. 17: eine isometrische Darstellung einer Handgriffvorrichtung gemäß einem weiteren vorteilhaften Ausführungsbeispiel, in die die Platine nach Fig. 16 eingesetzt ist,
- Fig. 18: eine isometrische Darstellung einer Handgriffvorrichtung gemäß einem weiteren vorteilhaften Ausführungsbeispiel, in die eine Videokamera integriert ist, und
- Fig. 19: eine isometrische Darstellung der Handgriffvorrichtung nach Fig. 18, wobei das Gehäuse des Sensormoduls nun nicht mehr transparent dargestellt ist.

Die Figuren sind lediglich schematischer Natur und dienen ausschließlich dem Verständnis der Erfindung. Die gleichen Elemente sind mit denselben Bezugszeichen versehen. Die Merkmale der unterschiedlichen Ausführungsbeispiele können auch frei miteinander kombiniert werden.

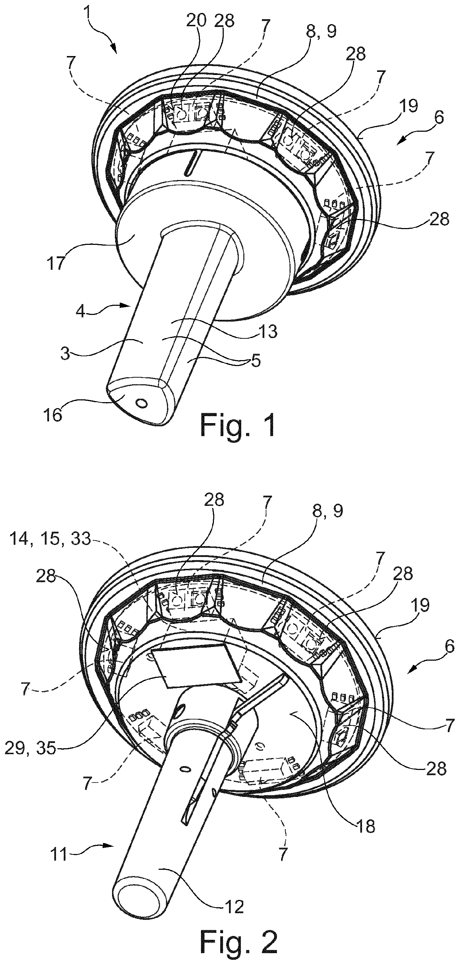

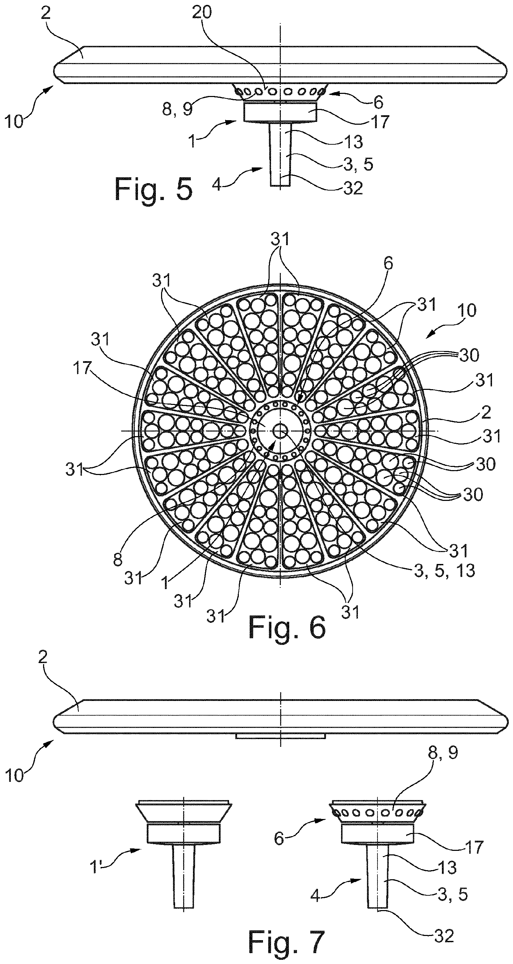

In Fig. 1 ist zunächst eine erfindungsgemäße Handgriffvorrichtung 1 nach einem vorteilhaften ersten Ausführungsbeispiel gut zu erkennen. Die Handgriffvorrichtung 1 ist für die Montage / für das vorübergehende Anbringen an einer nachfolgend in den Fign. 5 bis 9 sowie 11 bis 13 näher beschriebenen Operationsleuchte 10 vorbereitet. Die Erfindung betrifft somit nicht nur die Handgriffvorrichtung 1 selbst, sondern auch eine Operationsleuchte 10 mit einer solchen Handgriffvorrichtung 1.

Die Handgriffvorrichtung 1 weist ein, zum Anbringen an einem Leuchtenaufnahmekörper 2 der Operationsleuchte 10 vorbereitetes Griffelement 3 auf, welches Griffelement 3 auch schlicht als Handgriff bezeichnet ist. Das Griffelement 3 ist folglich derart dimensioniert, dass es durch eine Hand einer Person, wie einem Operateur, gegriffen werden kann, um die in einem Betriebszustand der Handgriffvorrichtung 1, in dem sie mit dem Leuchtenaufnahmekörper 2 der Operationsleuchte 10 fest verbunden ist, in die gewünschte Position zu bringen. Die nachfolgend (z.B. in Verbindung mit Fign. 5 und 6) noch näher beschriebene Operationsleuchte 10 weist weiterhin eine hier der Übersichtlichkeit halber nicht dargestellte Tragstruktur auf, die mit dem Leuchtenaufnahmekörper 2 verbunden ist und wodurch der Leuchtenaufnahmekörper 2 beliebig positionierbar ist.

An einem Außenbereich 4 des Griffelementes 3 ist eine Grifffläche 5 ausgebildet, die der Operateur im Betrieb zum Positionsändern der Operationsleuchte 10 berührt. Das Griffelement 3 an sich weist einen stabähnlichen, hohl ausgebildeten Hülsenabschnitt 13 auf, der sich länglich erstreckt. Unmittelbar an dessen Außenumfangseite / Außenmantelseite ist jene Grifffläche 5 ausgebildet. Das Griffelement 3 ist an seinem Außenbereich 4 derart ausgebildet, dass es eine möglichst glatte Oberfläche / Grifffläche 5 aufweist, die leicht sterilisierbar ist. Das heißt, die Rauhigkeit der Grifffläche 5 ist derart gewählt, dass eine Reinigung mit anschließendem Sterilisieren einfach durchführbar ist und ein Fangen von Schmutzpartikeln vermieden ist.

Der Hülsenabschnitt 13 weist an einer Stirnseite, die im Betrieb dem Leuchtenaufnahmekörper 2 abgewandt ist, einen Deckel 16 auf, der das Innere des Hülsenabschnittes 13 zur Umgebung hin schützt / abschließt. Der Deckel 16 bildet daher einen ersten axialen Endbereich des Hülsenabschnittes 13 des Griffelementes 3 aus. Mit einem zweiten, dem ersten Endbereich gegenüberliegenden Endbereich geht der Hülsenabschnitt 13 in einen scheibenförmigen Verbreiterungsabschnitt 17 über. Verbreiterungsabschnitt 17 sowie Hülsenabschnitt 13 sind stoffeinteilig miteinander ausgeführt, d.h. einstückig miteinander verbunden.

Wiederum mit dem Griffelement 3 ist ein erfindungsgemäßes Sensormodul 6 verbunden. Das Sensormodul 6 ist wiederabnehmbar mit dem Griffelement 3 verbunden. Dabei ist das Sensormodul 6 form- und kraftschlüssig mit dem Griffelement 3 verbunden. Das Sensormodul 6, das gesamtheitlich in Fig. 2 in einem vom Griffelement 3 demontierten Zustand zu erkennen ist, weist wiederum ein Gehäuse 8 auf. Das Gehäuse 8 weist wiederum einen scheibenförmigen Hohlkörperabschnitt 9 auf bzw. ist durch diesen scheibenförmigen Hohlkörperabschnitt 9 ausgebildet. Der scheibenförmige Hohlkörperabschnitt 9 ist zur Umgebung hin abgedichtet. Der Hohlkörperabschnitt 9 ist zum einen durch zwei voneinander beabstandete Wände - einer Vorderwand 18 und einer Rückwand 19 - ausgebildet. Zum anderen weist der Hohlkörperabschnitt 9 eine ringförmig verlaufende Seitenwand 20 auf, die die Vorderwand 18 mit der Rückwand 19 verbindet. Die Vorderwand 18 ist dabei jene flach verlaufende Wand des Hohlkörperabschnittes 9, die im Betrieb dem Griffelement 3 zugewandt ist, wohingegen die Rückwand 19 Gehäuses jene flach verlaufende Wand des Hohlkörperabschnittes 9 ist, die im Betrieb dem Griffelement 3 abgewandt ist.

Weiterhin, weist das Sensormodul 6, neben dem Gehäuse 8, einen Handgriffaufnahmeabschnitt 11 auf, der mit dem Gehäuse 8 / dem Hohlkörperabschnitt 9 fest verbunden ist. Dieser Handgriffaufnahmeabschnitt 11 ist dabei mit der Vorderwand 18 des Hohlkörperabschnittes 9 fest verbunden. Der Handgriffaufnahmeabschnitt 11 bildet einen Aufnahmezapfen 12 aus, der sich senkrecht von der, sich in einer gedachten Erstreckungsebene erstreckenden Vorderwand 18 des Hohlkörperabschnittes 9 weg erstreckt. Eine Längsachse 32 des Aufnahmezapfens 12 verläuft folglich senkrecht zu der Vorderwand 18 bzw. zu deren Erstreckungsebene.

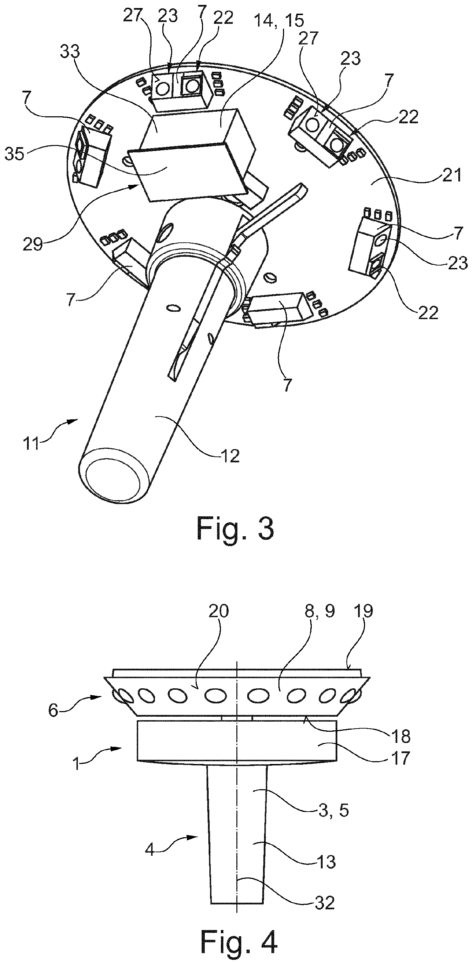

Der Aufnahmezapfen 12 ist derart dimensioniert und auf den Hülsenabschnitt 13 des Griffelementes 3 abgestimmt, dass dieser in den Hülsenabschnitt 13 einschiebbar ist. In dem in Fig. 1 dargestellten zusammengebauten Zustand der Handgriffvorrichtung 1 ist dieser Aufnahmezapfen 12 form- und kraftschlüssig in den Hülsenabschnitt 13 eingeschoben. Das Griffelement 3 ist daher form- und kraftschlüssig mit dem Sensormodul 6 verbunden. Wie auch in der schematischen Darstellung nach Fig. 4 zu erkennen ist, ist der Verbreiterungsabschnitt 17 in diesem zusammengebauten Zustand in axialer Richtung des Aufnahmezapfens 12 (entspricht axialer Richtung der Längsachse 32 bzw. des Hülsenabschnittes 13) von der Vorderwand 18 distanziert gehalten.

In Fig. 3 ist schließlich der innere Aufbau des Sensormoduls 6 veranschaulicht. In Fig. 3 ist der Übersichtlichkeit halber gegenüber der Fig. 2 das Gehäuse 8 / der Hohlkörperabschnitt 9 weggelassen, wodurch besonders gut die elektronische Einheit des Sensormoduls 6 zu erkennen ist. Auf einer scheibenförmigen Platine 21 dieser elektronischen Einheit sind erfindungsgemäß mehrere Abstandssensoren 7 positioniert. Diese Abstandssensoren 7 sind alle gleich ausgebildet und funktionierend.

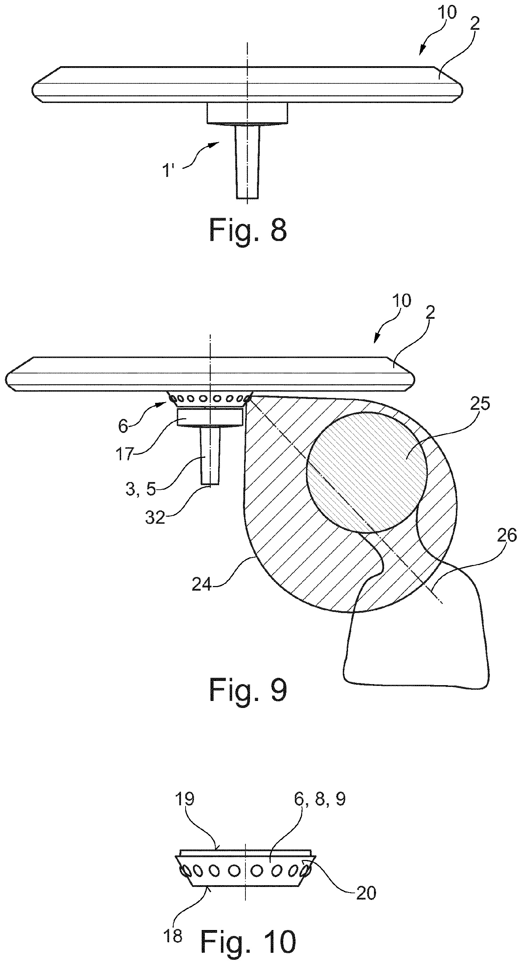

Die Abstandssensoren 7 sind als Infrarotsensoren (auch als Infrarotdistanzsensoren bezeichnet) ausgebildet. Jeder Abstandssensor 7 weist dabei einen im Wesentlichen tropfenförmigen / ballonförmigen Wirkbereich 24 auf, innerhalb dessen ein Objekt 25, wie ein Kopf des Operateurs detektierbar ist. Mit einem Infrarotsender 22 des Abstandssensors 7 wird hierfür Infrarotlicht ausgesandt, welches Infrarotlicht sich im Wesentlichen trichter-/kegelförmig entlang einer gedachten Richtachse 26 räumlich ausbreitet. Neben dem Infrarotsender 22 weist der Abstandssensor 7 auch einen Infrarotnehmer 23 auf. Der Infrarotnehmer 23 ist so ausgelegt und ausgebildet, dass er, wenn sich ein Objekt 25 innerhalb des Wirkbereiches 24 befindet, einen durch das Objekt 25 reflektierten Anteil des zuvor durch den Infrarotsender 22 ausgesandten Infrarotlicht messtechnisch erfasst / detektiert. Der Wirkbereich 24 ist besonders gut in Fig. 9 zu erkennen, wobei in dieser Abbildung die Handgriffvorrichtung 1 bereits an der Operationsleuchte 10 montiert ist. Der an dem Objekt 25 reflektierte Anteil an Infrarotlicht wird daher dem Infrarotnehmer 23 zugeführt, der das reflektierte Infrarotlicht in ein Steuersignal umsetzt. In Abhängigkeit der Entfernung des Objektes 25 vom Abstandssensor 7 / vom Infrarotnehmer 23, detektiert der Infrarotnehmer 23 das Signal zu einem früheren oder einem späteren Zeitpunkt relativ zu dem Absendezeitpunkt des Infrarotlichtes durch den Infrarotsender 22. Dadurch ist der Abstand zwischen dem Objekt 25 und der Handgriffvorrichtung 1 einfach detektierbar. Der Wirkbereich 24 ist folglich durch die Form des ausgesandten Infrarotlichtes sowie die Reichweite des Infrarotnehmers 23 beschränkt. Jeder Abstandssensor 7 ist daher ausgebildet, eine Position des Objektes 25 in einem Bereich zwischen dem Lampenaufnahmegehäuse 2 und einem Wundfeld über den Abstand des Objektes 25 relativ zu dem Abstandssensor 7, bzw. zu dem Sensormodul 6, zu detektieren / zu ermitteln.

Die Abstandssensoren 7 sind auf der Platine 21 derart angebracht, dass ihr Wirkbereich 24 mit seiner Richtachse 26 quer / schräg zu der Längsachse 32 des Aufnahmezapfens 12, insbesondere vorzugsweise um etwa 45° versetzt zu dieser Längsachse 32 ausgerichtet ist. Der Infrarotsender 22 und der Infrarotnehmer 23 sind in einer Aufnahmeebene 27 angeordnet / ausgerichtet, die senkrecht zu der Richtachse 26 ausgerichtet ist.

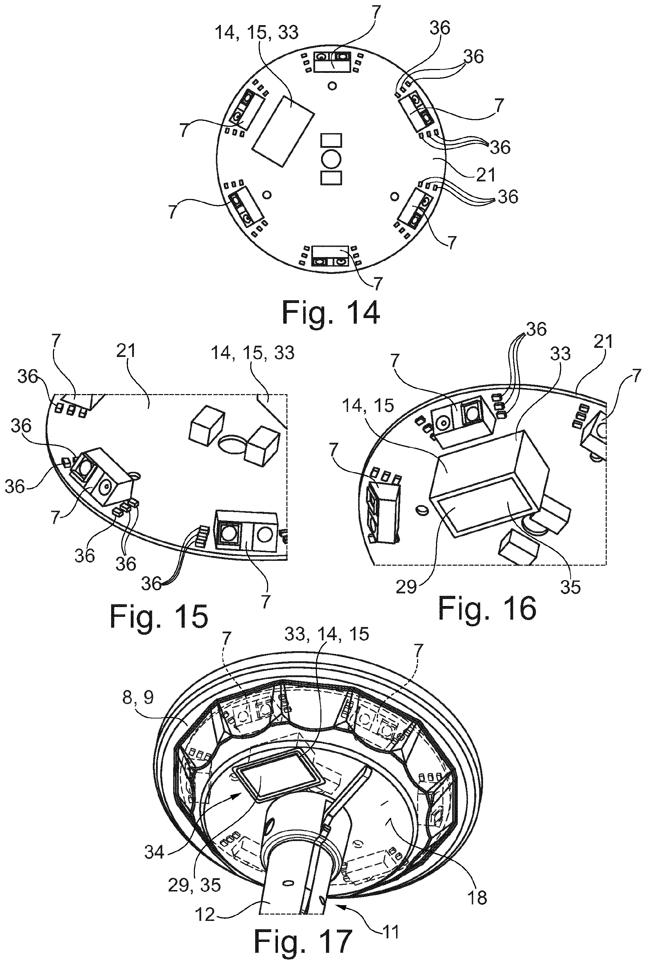

In dieser Ausführungsform sind sechs Abstandssensoren 7 im Wesentlichen entlang einer kreisförmigen, gedachten Umfangslinie um die Längsachse des Aufnahmezapfens 12 herum verteilt angeordnet, wobei jedoch auch in weiteren Ausführungsformen andere Stückzahlen von Abstandssensoren 7 gewählt sind, bspw. weniger oder mehr als sechs, vorzugsweise sieben, acht, neun oder mindestens zehn Abstandssensoren 7. In der Fig. 14 ist auch nochmals die Verteilung der Abstandssensoren 7 schematisch dargestellt, wobei die Rechnereinheit 14 in dieser Ansicht der Übersichtlichkeit halber kein Mikrofon 29 aufweist. Die Abstandssensoren 7 sind jeweils in gleichen Abständen entlang der gedachten Umfangslinie angeordnet und haben einen im Wesentlichen gleichen Abstand zu der Längsachse 32 des Aufnahmezapfens 12. Die Abstandssensoren 7 sind dabei derart angeordnet und ausgerichtet, dass sie mit ihren Wirkbereichen 24 die Position eines Objektes 25 innerhalb des gesamten Umfangs, d.h. in einem Winkelbereich von 360° um die Längsachse 32 herum, erfassen können.

Wie besonders gut auch in den Fign. 1 und 2 zu erkennen ist, ist die Seitenwand 20 des Gehäuses 8 mit mehreren Abdeckungsbereichen 28 ausgebildet. Jeder Abdeckungsbereich 28 erstreckt sich parallel zu der Aufnahmeebene 27 und daher konisch zwischen der Vorderwand 18 und der Rückwand 19. Die kreisrunde Vorderwand 18 weißt einen geringeren Durchmesser auf als die ebenfalls kreisrund ausgebildete Rückwand 19. Die Seitenwand 20 ist in dieser Ausführung aus einem Material hergestellt, das durchlässig für die Messsignale der Abstandssensoren 7 ist. Folglich ist diese Seitenwand 20 durchlässig für Infrarotlicht. Da das konisch ausgeformte Gehäuse 8 / der konisch ausgeformte Hohlkörperabschnitt 9 gesamtheitlich aus dem gleichen Werkstoff besteht, ist dieser gesamtheitlich aus einem Infrarotlichtdurchlässigen Material / Werkstoff ausgebildet. Neben der infrarotdurchlässigen Seitenwand 20, ist somit auch die Vorderwand 18 infrarotlichtdurchlässig. Jedem Abstandssensor 7 ist folglich ein parallel zu dessen Aufnahmeebene 27 verlaufender Abdeckungsbereich 28 zugeordnet, der sich im Wesentlichen um die gleiche Breite erstreckt, wie der jeweilige Abstandssensor 7. Jeder der Abstandssensoren 7 ist weiterhin mit einer auf der Platine 21 angeordneten Rechnereinheit 14 elektronisch verbunden. Diese Rechnereinheit 14 ist insbesondere dafür ausgestaltet, in einem im Betrieb mit der Operationsleuchte 10 verbundenen Zustand Steuersignale an die Operationsleuchte 10 bzw. an dessen (zentrale) Steuereinheit, die hier der Übersichtlichkeit halber nicht weiter dargestellt ist, zu übertragen, so dass entsprechend der ermittelten Position des Objektes 25 die einzelnen Leuchten / Einzelleuchten 30 der Leuchtenfelder 31 der Operationsleuchte 10, die das Objekt 25 bescheinen, abgedimmt oder entsprechend abgeschaltet werden können.

Im Weiteren ist in dieser Rechnereinheit 14, nämlich in einem Elektronikgehäuse 33, der die Rechnereinheit 14 aufnimmt, eine Sprachsteuerungseinheit 15 integriert. Die Sprachsteuerungseinheit 15 ist wiederum elektrisch mit der Rechnereinheit 14 verbunden. Folglich können durch ein Mikrofon 29 der Sprachsteuerungseinheit 15 aufgenommene Schallsignale in ein entsprechendes Steuersignal mittels der Rechnereinheit 14 umgewandelt werden und in einem an der Operationsleuchte 10 angebrachten Zustand an deren zentrale Steuereinheit wiederum übermittelt werden. In Fig. 3 überragt eine schalldurchlässige Außenabdeckung des Mikrofons 29 zu allen Seiten hin das Elektronikgehäuse 33. Dadurch ist die Operationsleuchte 10 auch individuell mit einzelnen Sprachbefehlen bedienbar. Neben der Bedienung der Helligkeit / Beleuchtungsstärke der jeweiligen Einzelleuchten 30 der Operationsleuchte 10, ist es auch möglich bspw. eine Stellung der Operationsleuchte 10 mittels dieser Sprachbefehle zu verändern. Die Sprachsteuerungseinheit 15 weist auch einen Lautsprecher auf.

Wie in Fig. 2 gut zu erkennen ist, weist die Vorderwand 18 des Hohlkörperabschnittes 9 eine entsprechende Aussparung auf, in die die Membran / Außenabdeckung 35 des Mikrofons 29, die schallübertragend ausgebildet ist, eingesetzt ist. Folglich ist eine Schallübertragung durch das Gehäuse 8 in den Innenraum hinein ermöglicht.

In einer weiteren Ausführungsform ist die Recheneinheit 14 nicht wie hier kabelgebunden mit der zentralen Steuereinheit der Operationsleuchte 10 verbunden, sondern drahtlos, vorzugsweise über Bluetooth.

In den Fign. 5 und 6 ist dann besonders gut auch eine erfindungsgemäße Operationsleuchte 10 mit einer montierten Handgriffvorrichtung 1 gemäß der Fign. 1 bis 4 zu erkennen. Die Operationsleuchte 10 weist dafür den Leuchtenaufnahmekörper 2, der auch als Grundkörper bezeichnet ist, auf, in dem mehrere Einzelleuchten 30 eingesetzt sind. Die einzelnen Einzelleuchten 30 sind in dieser Ausführungsform mit weiteren Einzelleuchten 30 zu unterschiedlichen Leuchtenfeldern 31 kombiniert / gruppiert. Die Operationsleuchte 10 weist in dieser Ausführung einen im Wesentlichen scheibenförmigen und gehäuseartigen Leuchtenaufnahmekörper 2 auf, der in weiteren Ausführungen jedoch auch andere Ausgestaltungen aufweist und bspw. auch mehrteilig, d.h. aus mehreren Leuchtenaufnahmekörper-Segmenten, ausbildbar ist. Die Leuchtenfelder 31 (auch als Leuchtfelder bezeichnet) sind jeweils im Wesentlichen gleich funktionierend, angesteuert und aufgebaut.

Jedes Leuchtenfeld 31 weist die gleiche Anzahl an Einzelleuchten 30 auf. Die Einzelleuchten 30 eines Leuchtenfeldes 31 variieren dabei in ihrer Größe und/oder Helligkeit / Leuchtstärke / Beleuchtungsstärke. Auch die Leuchtfarbe der Einzelleuchten 30 untereinander variiert hierbei. Jedes Leuchtenfeld 31 ist dabei in Form eines tortenförmigen Stückes der sich scheibenförmig um die zentrale Handgriffvorrichtung 1 herum erstreckenden Gesamtzahl von Einzelleuchten 30 ausgebildet. Jede der Einzelleuchten 30 umfasst dabei ausschließlich eine LED, in weiteren Ausführungen jedoch auch mehrere LEDs. Jede Einzelleuchte 30 weißt eine der LED zugeordnete Linse / Lindenoptik auf. Jede der Einzelleuchten 30 ist dabei mit der zentralen Steuereinheit der Operationsleuchte 10 elektrisch verbunden und in Abhängigkeit der Steuersignale seitens der zentralen Steuereinheit unabhängig voneinander regelbar, insbesondere in ihrer Leuchtstärke / Leuchtfarbe regelbar.

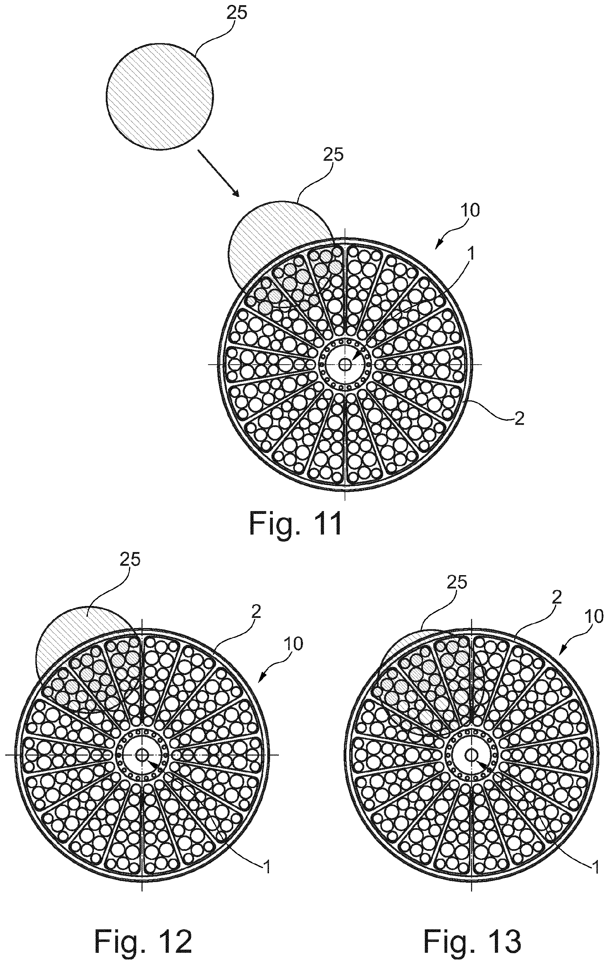

Wie im Zusammenhang mit den Fign. 9 und 11 bis 13 zu erkennen ist, ist im Betrieb der Operationsleuchte 10 ein Überwachen eines Bereiches, der durch die Einzelleuchten 30 ausgeleuchtet wird, mittels der Abstandssensoren 7 umgesetzt. Die einzelnen Abstandssensoren 7 wirken dabei vorzugsweise in einer Reichweite von mehr als einem, vorzugsweise von mehr als zwei Metern in Richtung der Richtachse 26. Dadurch wird aufgrund eines in den Lichtstrahl / in das Lichtstrahlbündel einer Einzelleuchte 30 tretenden Objektes 25 gemäß der Fign. 11 bis 13 durch die Abstandssensoren 7 der Abstand zwischen dem Objekt 25 und dem Sensormodul 6 erfasst / detektiert und daraufhin ein Steuersignal durch die Rechnereinheit 14 erzeugt, wodurch mittels der zentralen Steuereinheit die Einzelleuchten 30 der betroffenen Leuchtenfelder 31 (d.h. die Einzelleuchten 30, die mit ihren Lichtstrahlbündel das Objekt direkt beleuchten) herunter gedimmt bzw. abgeschaltet werden. Ein Abdimmen erfolgt hierbei je nach Höhenabstand zwischen der Operationsleuchte 10 und dem Objekt 25. Wie in den Fign. 11 bis 13 gut zu erkennen ist, können dabei die Leuchtenfelder 31 gemäß Fig. 13 gesamtheitlich oder die Einzelleuchten 30 der Leuchtenfelder 31 einzeln, je nach Position des Objektes 25 relativ zu der Handgriffvorrichtung 1 / zum Abstandssensor 7, abgeschaltet / gedimmt werden.

Zur Anzeige ob ein Objekt 25 in dem Wirkbereich 24 des jeweiligen Abstandssensors 7 befindlich ist, sind weiterhin auf der Platine 21 mehrere Anzeigeleuchten 36 je Abstandssensor 7 angebracht, die besonders gut in Fig. 15 zu erkennen sind. Wenn ein Objekt 25 in dem Wirkbereich 24 des jeweiligen Abstandssensors 7 befindlich ist, erhöht sich die Anzahl an leuchtenden / eingeschalteten Anzeigeleuchten 36 mit einem sich verringernden Abstand zwischen Abstandssensor 7 und Objekt 25. In dieser Ausführung sind je Abstandssensors 7 sechs Anzeigeleuchten 36 vorhanden. Jede Anzeigeleuchte 36 ist als eine LED ausgebildet. Auch ist es möglich in Abhängigkeit des Abstandes zwischen Abstandssensor 7 und Objekt 25 zwischen verschiedenen Anzeigeleuchte 36, die sich in ihrer Leuchtfarbe unterscheiden, hin- und herzuschalten.

Die Handgriffvorrichtung 1 ist in ihrem montierten Zustand zentral an dem Leuchtenaufnahmekörper 2 befestigt und somit mit der Längsachse 32 koaxial zu einer gedachten Mittelachse des Leuchtenaufnahmekörpers 2 angeordnet.

Im Weiteren hat auch das Mikrofon 29 eine Reichweite von mindestens einem, besonders bevorzugt von mindestens zwei Metern, wodurch Schallquellen, die ein entsprechendes Schallsteuersignal erzeugen, etwa das Objekt 25 selbst, detektiert werden können, um die Operationsleuchte 10 entsprechend anzusteuern.

In den Fign. 7 und 8 wird dann nochmals besonders gut die vorteilhafte Ausbildung der Handgriffvorrichtung 1 sowie die Zusammenwirkung mit der Operationsleuchte 10 erkennbar. So ist es ein Einfaches, eine herkömmliche Handgriffvorrichtung 1' nach dem Stand der Technik, wie sie in Fig. 8 bzw. auf der linken Seite in Fig. 7 dargestellt ist, durch eine erfindungsgemäße Handgriffvorrichtung 1, wie sie auf der rechten Seite in Fig. 7 dargestellt ist, auszutauschen, indem der wieder abnehmbare Teil der alten Handgriffvorrichtung 1' von der Operationsleuchte 10 entfernt wird und die erfindungsgemäße Handgriffvorrichtung 1 an dem Leuchtenaufnahmekörper 2 befestigt wird. Zu diesem Zwecke bildet das Sensormodul 6 an der Rückwand 19 des Hohlkörperabschnittes 9 einen wiederabnehmbaren Abschnitt aus, der wiederabnehmbar mit dem Leuchtenaufnahmekörper 2 verbunden wird. Zu diesem Zwecke ist der Hohlkörperabschnitt 9 form- und kraftschlüssig, vorzugsweise mittels einer Art Bajonettverschluss wiederabnehmbar / lösbar mit dem Leuchtenaufnahmekörper 2 verbunden. Dadurch ist dann die fertig montierte Stellung der Operationsleuchte 10 bzw. der Handgriffvorrichtung 1 umgesetzt, wie es etwa in Fig. 9 dargestellt ist. Auch ist in dem Sensormodul 6 weiter eine hier der Übersichtlichkeit halber nicht weiter dargestellte Bildaufzeichnungseinrichtung integriert, die zumindest eine Videokamera zur Aufnahme des Wundfeldes aufweist und durch die Sprachsteuerungseinheit 15 steuerbar ist.

In Verbindung der Fign. 16 und 17 ist ein weiteres Ausführungsbeispiel der erfindungsgemäßen Handgriffvorrichtung 1 schematisch dargestellt, wobei diese Handgriffvorrichtung 1 gemäß dem ersten Ausführungsbeispiel funktioniert und ausgebildet ist. Als Unterschied ist hier die Außenabdeckung 35 des Mikrofons 29 in einen durch das Elektronikgehäuse 33 gebildeten Rahmen eingesetzt und dadurch besonders geschickt formschlüssig in dem Elektronikgehäuse 33 gehalten. In Fig. 17 ist dabei zu erkennen, dass in dem Gehäuse 8 des Sensormoduls 6, nämlich in dessen Vorderwand 18, eine mit dem Elektronikgehäuse 33 korrespondierende Aussparung 34 ausgebildet ist, in die das Elektronikgehäuse 33 seitens der Außenabdeckung 35 hineinragt.

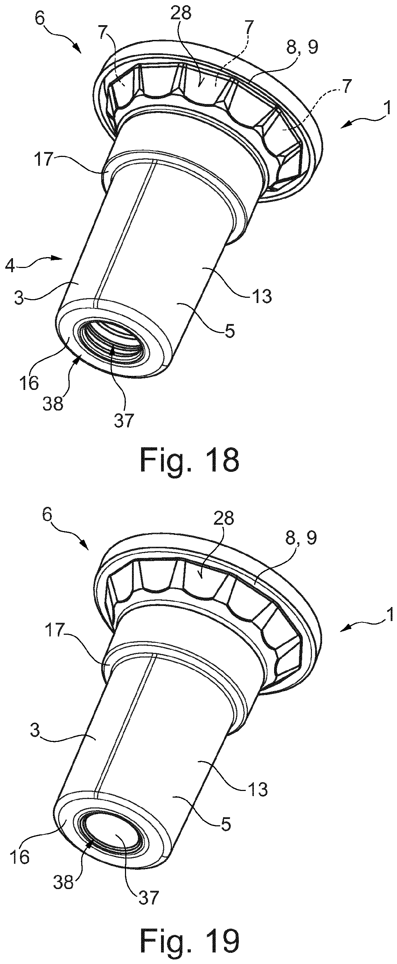

In den Fign. 18 und 19 ist ebenfalls noch ein weiteres Ausführungsbeispiel der erfindungsgemäßen Handgriffvorrichtung 1 dargestellt, wobei diese Handgriffvorrichtung 1 gemäß dem ersten Ausführungsbeispiel funktioniert und ausgebildet ist. Insbesondere ist hierbei jedoch das Griffelement 3 dicker ausgebildet. Dies liegt daran, dass in dem Griffelement 3 nun eine Videokamera 37 zur Aufnahme des Wundfeldes angeordnet ist. Von der Videokamera 37 ist die äußerte Scheibe / das äußerste Glas des Objektives zu erkennen, wobei die Lichteintrittsachse der Videokamera 37 konzentrisch zu einem Durchgangsloch 38 ausgebildet ist. Die Videokamera 37 ist von der Grifffläche 5 umgeben, jedoch im Bereich des Deckels 16 mit dem Durchgangsloch 38 versehen, durch die die Videokamera 37 das Äußere der Handgriffvorrichtung 1 erfasst.

In Verbindung mit Fig. 10 ist dann noch ein weiteres Ausführungsbeispiel der erfindungsgemäßen Handgriffvorrichtung offenbart, wobei in erster Linie ein weiteres Sensormodul 6 in einer alternativen Ausführung zu erkennen ist. Das Sensormodul 6 besteht hierbei nur aus dem zuvor beschriebenen, scheibenförmigen Hohlkörperabschnitt 9 in Form eines Zwischenrings, der das Gehäuse 8 ausbildet. Ein als Aufnahmezapfen 12 ausgebildeter Handgriffaufnahmeabschnitt 11 ist hierbei weggelassen. In dieser Ausführungsform ist es umgesetzt, dass eine bestehende Handgriffvorrichtung 1 direkt mit dem Gehäuse 8 wieder abnehmbar verbunden ist. Auch dadurch ist es daher dann möglich, ein wieder abnehmbares Sensormodul 6 zur Verfügung zu stellen. Dessen Aufbau sowie Funktionsweise entspricht hierbei dem Sensormodul 6 des ersten Ausführungsbeispieles.

In anderen Worten ausgedrückt, ist eine Handgriffvorrichtung 1 umgesetzt, die als zentrale Handgriffeinheit ausgebildet ist. Die Sensoren in Form der Abstandssensoren 7 sind in dieser zentralen Handgriffeinheit 1 in der OP-Leuchte / Operationsleuchte 10 angeordnet. Von hier aus haben die Sensoren 7 eine optimale "Sicht" (Sichtbereich entspricht Wirkbereich 24) auf die Köpfe 25 der Anwender. Die Sensoren 7 erfassen den Kopf 25 als Hindernis und blenden die dahinterliegenden Teilbereiche der Leuchtenfelder 31 / die entsprechenden Einzelleuchten 30 aus. Die Sensoren 7 sind auf einer ringförmigen Platine 21 bestückt. Das runde / konische Bauteil, nämlich die Seitenwand 20, ist transparent (IR-durchlässig) ausgeführt.

### Bezugszeichen

- 1: Handgriffvorrichtung
- 1': Handgriffvorrichtung nach dem Stand der Technik
- 2: Leuchtenaufnahmekörper
- 3: Griffelement
- 4: Außenbereich
- 5: Grifffläche
- 6: Sensormodul
- 7: Abstandssensor
- 8: Gehäuse
- 9: Hohlkörperabschnitt
- 10: Operationsleuchte
- 11: Handgriffaufnahmeabschnitt
- 12: Aufnahmezapfen
- 13: Hülsenabschnitt
- 14: Recheneinheit
- 15: Sprachsteuerungseinheit
- 16: Deckel
- 17: Verbreiterungsabschnitt
- 18: Vorderwand
- 19: Rückwand
- 20: Seitenwand
- 21: Platine
- 22: Infrarotsender
- 23: Infrarotnehmer
- 24: Wirkbereich
- 25: Objekt
- 26: Richtachse
- 27: Aufnahmeebene
- 28: Abdeckungsbereich
- 29: Mikrofon
- 30: Einzelleuchte
- 31: Leuchtenfeld
- 32: Längsachse
- 33: Elektronikgehäuse
- 34: Aussparung
- 35: Außenabdeckung
- 36: Anzeigeleuchte

## Patentansprüche

1. Handgriffvorrichtung (1), die zum Anbringen an einer Operationsleuchte (10) vorbereitet ist, mit einem Griffelement (3), das an einem Außenbereich (4) eine Grifffläche (5) ausbildet, **dadurch gekennzeichnet, dass** mit dem Griffelement (3) ein Sensormodul (6) wieder abnehmbar verbunden ist, wobei das Sensormodul (6) mehrere Abstandssensoren (7) aufweist, die jeweils zum Detektieren einer Position eines Objektes ausgelegt und mit einer Richtachse (26) ihres Wirkbereiches (24) voneinander weggerichtet sind.

2. Handgriffvorrichtung (1) nach Anspruch 1, **dadurch gekennzeichnet, dass** das Sensormodul (6) ein Gehäuse (8) aufweist, in dem die Abstandssensoren (7) aufgenommen sind.

3. Handgriffvorrichtung (1) nach Anspruch 2, **dadurch gekennzeichnet, dass** das Gehäuse (8) einen scheibenförmigen Hohlkörperabschnitt (9) aufweist, innerhalb dessen die Abstandssensoren (7) angeordnet sind.

4. Handgriffvorrichtung (1) nach Anspruch 2 oder 3, **dadurch gekennzeichnet, dass** das Gehäuse (8) im Bereich des jeweiligen Abstandssensors (7) durchlässig für ein durch den jeweiligen Abstandssensor (7) zu detektierendes Messsignal ausgebildet ist.

5. Handgriffvorrichtung (1) nach einem der Ansprüche 1 bis 4, **dadurch gekennzeichnet, dass** der jeweilige Abstandssensor (7) als ein Infrarotsensor ausgestaltet ist.

6. Handgriffvorrichtung (1) nach einem der Ansprüche 1 bis 5, **dadurch gekennzeichnet, dass** das Sensormodul (6) einen Handgriffaufnahmeabschnitt (11) aufweist, der wiederabnehmbar mit dem Griffelement (3) verbunden ist.

7. Handgriffvorrichtung (1) nach Anspruch 6, **dadurch gekennzeichnet, dass** der Handgriffaufnahmeabschnitt (11) einen Aufnahmezapfen (12) ausbildet, auf dem in zumindest einem Betriebszustand der Handgriffvorrichtung (1) ein, die Grifffläche (5) aufweisender Hülsenabschnitt (13) des Griffelementes (3) aufgesteckt ist.

8. Handgriffvorrichtung (1) nach einem der Ansprüche 1 bis 7, **dadurch gekennzeichnet, dass** das Griffelement (3) form- und/oder kraftschlüssig mit dem Sensormodul (6) in zumindest einem Betriebszustand verbunden ist.

9. Handgriffvorrichtung (1) nach einem der Ansprüche 1 bis 8, **dadurch gekennzeichnet, dass** das Sensormodul (6) eine Rechnereinheit (14) aufweist, die elektrisch mit dem jeweiligen Abstandssensor (7) verbunden ist und abhängig von den mittels des jeweiligen Abstandssensors (7) ermittelten Messdaten Steuersignale zur Steuerung der Operationsleuchte (10) erzeugt.

10. Handgriffvorrichtung (1) nach einem der Ansprüche 1 bis 9, **dadurch gekennzeichnet, dass** in dem Sensormodul (6) eine Sprachsteuerungseinheit (15) zum Betätigen des jeweiligen Abstandssensors (7) und/oder zum Steuern der Operationsleuchte (10) in einem Betriebszustand integriert ist.

11. Handgriffvorrichtung (1) nach Anspruch 9 und 10, **dadurch gekennzeichnet, dass** die Sprachsteuerungseinheit (15) elektronisch mit der Rechnereinheit (14) verbunden ist.

12. Operationsleuchte (10) mit einer Handgriffvorrichtung (1) nach einem der Ansprüche 1 bis 11, wobei die Handgriffvorrichtung (1) zumindest teilweise wieder abnehmbar mit einem Leuchtenaufnahmekörper (2) der Operationsleuchte (10) verbunden ist.

13. Operationsleuchte (10) nach Anspruch 12, **dadurch gekennzeichnet, dass** das Sensormodul (6) wieder abnehmbar an dem Leuchtenaufnahmekörper (2) angebracht ist.

14. Operationsleuchte (10) nach Anspruch 12 oder 13, **dadurch gekennzeichnet, dass** eine Rechnereinheit (14) des Sensormoduls (6) elektrisch mit einer zentralen Steuereinheit der Operationsleuchte (10) verbunden ist.

## Claims

1. A handle device (1) which is prepared for being arranged on a surgical light (10), comprising a grip element (3) which forms a grip surface (5) at an outer area (4), **characterized in that** a sensor module (6) is detachably connected to the grip element (3), wherein the sensor module (6) includes a pluraltiy of distance sensors (7) each of which is designed for detecting a position of an object and are directed away from each other with central axes (26) of their operating range (24).

2. The handle device (1) according to claim 1, **characterized in that** the sensor module (6) includes a housing (8) in which the distance sensors (7) are accommodated.

3. The handle device (1) according to claim 2, **characterized in that** the housing (8) includes a disk-shaped cavity portion (9) inside of which the distance sensors (7) are arranged.

4. The handle device (1) according to claim 2 or 3, **characterized in that** the housing (8) within the area of the respective distance sensor (7) is configured to be permeable to a measuring signal that is to be detected by the respective distance sensor (7).

5. The handle device (1) according to any one of claims 1 to 4, **characterized in that** the respective distance sensor (7) is configured as an infrared sensor.

6. The handle device (1) according to any one of claims 1 to 5, **characterized in that** the sensor module (6) includes a handle receiving portion (11) which is detachably connected to the grip element (3).

7. The handle device (1) according to claim 6, **characterized in that** the handle receiving portion (11) forms a receiving journal (12) onto which a sleeve portion (13) of the grip element (3) including the grip surface (5) is attached in at least one operating condition of the handle device (1).

8. The handle device (1) according to any one of claims 1 to 7, **characterized in that** the grip element (3) is connected to the sensor module (6) via a form-fitting and/or a load-carrying connection in at least one operating condition.

9. The handle device (1) according to any one of claims 1 to 8, **characterized in that** the sensor module (6) includes a computer unit (14) which is electrically connected to the respective distance sensor (7) and generates control signals for controlling the surgical light (10) in response to the measuring data established by way of the respective distance sensor (7).

10. The handle device (1) according to any one of claims 1 to 9, **characterized in that** a voice control unit (15) for operating the respective distance sensor (7) and/or for controlling the surgical light (10) in an operating condition is integrated in the sensor module (6).

11. The handle device (1) according to claims 9 and 10, **characterized in that** the voice control unit (15) is electronically connected to the computer unit (14).

12. A surgical light (10) comprising a handle device (1) according to any one of claims 1 to 11, wherein the handle device (1) is at least partially detachably connected to a lamp holding body (2) of the surgical light (10).

13. The surgical light (10) according to claim 12, **characterized in that** the sensor module (6) is detachably arranged on the lamp holding body (2).

14. The surgical light (10) according to claim 12 or 13, **characterized in that** the computer unit (14) of the sensor module (6) is electrically connected to a central control unit of the surgical light (10).

## Revendications

1. Dispositif de poignée (1) qui est préparé pour le montage au niveau d'une lampe de salle d'opération (10), avec un élément de préhension (3) qui réalise au niveau d'une zone extérieure (4) une surface de préhension (5), **caractérisé en ce qu'**avec l'élément de préhension (3) un module de capteur (6) est raccordé de manière amovible, dans lequel le module de capteur (6) présente plusieurs capteurs de distance (7) qui sont conçus respectivement pour la détection d'une position d'un objet et sont dirigés avec un axe de direction (26) de sa zone active (24) à l'opposé l'un de l'autre.

2. Dispositif de poignée (1) selon la revendication 1, **caractérisé en ce que** le module de capteur (6) présente un boîtier (8) dans lequel les capteurs de distance (7) sont reçus.

3. Dispositif de poignée (1) selon la revendication 2, **caractérisé en ce que** le boîtier (8) présente une section de corps creux en forme de disque (9) à l'intérieur de laquelle les capteurs de distance (7) sont agencés.

4. Dispositif de poignée (1) selon la revendication 2 ou 3, **caractérisé en ce que** le boîtier (8) est réalisé dans la zone du capteur de distance respectif (7) de manière perméable à un signal de mesure devant être détecté par le capteur de distance respectif (7).

5. Dispositif de poignée (1) selon l'une quelconque des revendications 1 à 4, **caractérisé en ce que** le capteur de distance respectif (7) est configuré en tant que capteur à infrarouge.

6. Dispositif de poignée (1) selon l'une quelconque des revendications 1 à 5, **caractérisé en ce que** le module de capteur (6) présente une section de réception de poignée (11) qui est raccordée de manière amovible à l'élément de préhension (3).

7. Dispositif de poignée (1) selon la revendication 6, **caractérisé en ce que** la section de réception de poignée (11) réalise un tenon de réception (12), sur lequel dans au moins un état de fonctionnement du dispositif de poignée (1) une section de douille (13) de l'élément de préhension (3) présentant la surface de préhension (5) est enfichée.

8. Dispositif de poignée (1) selon l'une quelconque des revendications 1 à 7, **caractérisé en ce que** l'élément de préhension (3) est raccordé par complémentarité de formes et/ou à force au module de capteur (6) dans au moins un état de fonctionnement.

9. Dispositif de poignée (1) selon l'une quelconque des revendications 1 à 8, **caractérisé en ce que** le module de capteur (6) présente une unité de calcul (14) qui est raccordée électriquement au capteur de distance respectif (7) et génère, en fonction des données de mesure déterminées au moyen du capteur de distance respectif (7), des signaux de commande pour la commande de la lampe de salle d'opération (10).

10. Dispositif de poignée (1) selon l'une quelconque des revendications 1 à 9, **caractérisé en ce que** dans le module de capteur (6) une unité de commande vocale (15) est intégrée pour l'actionnement du capteur de distance respectif (7) et/ou pour la commande de la lampe de salle d'opération (10) dans un état de fonctionnement.

11. Dispositif de poignée (1) selon les revendications 9 et 10, **caractérisé en ce que** l'unité de commande vocale (15) est raccordée électroniquement à l'unité de calcul (14).

12. Lampe de salle d'opération (10) avec un dispositif de poignée (1) selon l'une quelconque des revendications 1 à 11, dans laquelle le dispositif de poignée (1) est raccordé au moins partiellement de manière amovible à un corps de réception de lampe (2) de la lampe de salle d'opération (10).

13. Lampe de salle d'opération (10) selon la revendication 12, **caractérisée en ce que** le module de capteur (6) est monté de manière amovible au niveau du corps de réception de lampe (2).

14. Lampe de salle d'opération (10) selon la revendication 12 ou 13, **caractérisée en ce qu'**une unité de calcul (14) du module de capteur (6) est raccordée électriquement à une unité de commande centrale de la lampe de salle d'opération (10).
